(19) Europäisches Patentamt
European Patent Office
Office européen des brevets

(11) **EP 4 335 360 A1**

(12) **EUROPEAN PATENT APPLICATION**

(43) Date of publication:
**13.03.2024 Bulletin 2024/11**

(21) Application number: **22195047.0**

(22) Date of filing: **12.09.2022**

(51) International Patent Classification (IPC):
**A61B 5/022** (2006.01)     **A61B 5/024** (2006.01)
**A61B 5/00** (2006.01)     **G16H 50/30** (2018.01)

(52) Cooperative Patent Classification (CPC):
**A61B 5/02225; A61B 5/02438; A61B 5/7264;**
**A61B 5/7275; G16H 50/30;** A61B 5/0245;
A61B 5/0535; A61B 2505/03; G16H 20/10;
G16H 40/63; G16H 40/67; G16H 50/20; G16H 50/70

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **MUEHLSTEFF, Jens**
  **Eindhoven (NL)**
• **DAVIDOIU, Valentina-Geta**
  **Eindhoven (NL)**

(74) Representative: **Philips Intellectual Property &**
**Standards**
**High Tech Campus 52**
**5656 AG Eindhoven (NL)**

(54) **NON-INVASIVE BLOOD PRESSURE MEASUREMENT**

(57)     A method for adaptively scheduling non-invasive blood pressure measurement time intervals based on using a risk model to compute a risk of a patient suffering a pre-defined one or more adverse clinical events, for example within a pre-defined time window, and also based on a clinician risk assessment for a patient.

FIG. 3

EP 4 335 360 A1

**Description**

FIELD OF THE INVENTION

This invention relates to the field of non-invasive blood pressure monitoring.

BACKGROUND OF THE INVENTION

Arterial Blood Pressure (ABP) is a key physiological parameter relevant for medical diagnosis, prevention as well as therapy guidance.

The gold standard for blood pressure measurement is the invasive measurement. This uses intra-arterial cannulation. This provides the most accurate measurements, and allows for continuous measurements. However, it necessitates trained medical staff and for this reason is usually applied only in the highest acuity settings, where very close monitoring and real-time alarming is required.

Most blood pressure measurement is performed non-invasively. The most common non-invasive blood pressure measurement (NIBP) technique in patient monitoring is standard cuff-based ABP measurement using automated oscillometry. This provides intermittent measurements. Typically intervals are set at fixed or standardized time intervals in dependence upon patient condition. For example, a typical time interval might be every 15 minutes in an ICU setting. However, this leaves open a significant risk that a change in patient condition could occur suddenly during one of the intervals.

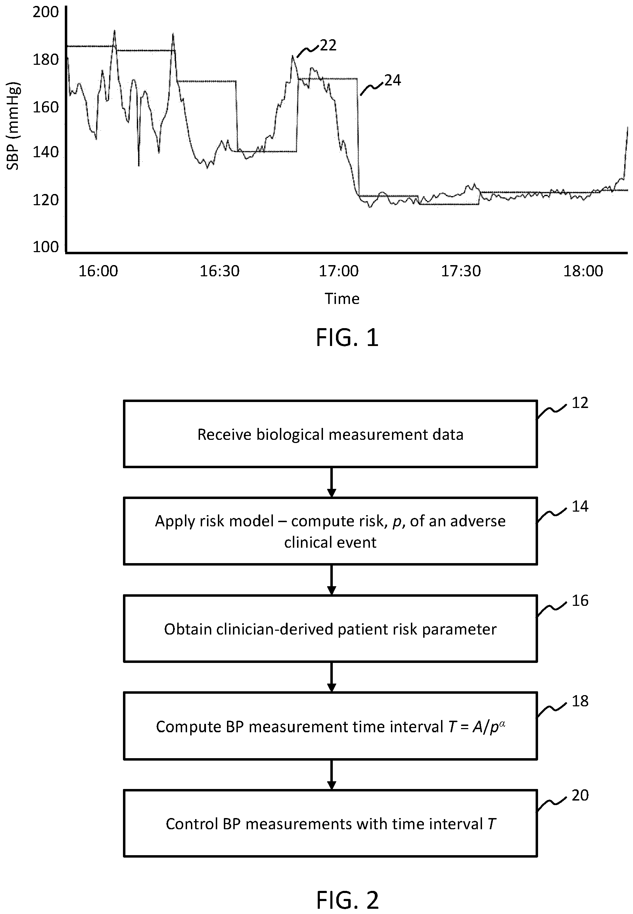

For example, Fig. 1 illustrates an example of a real-time systolic blood pressure (y-axis) of a patient as a function of time (trace 22), as compared with blood pressure measurements recorded by a measurement device at regular intervals (trace 24). The interval time is 15 minutes in this example. It can be seen from this example how, if the blood pressure drops suddenly during an inter-measurement interval, this could be missed for a significant time until the next measurement is taken. This is apparent for example within the interval from around 16:15 to around 16:30, where the patient's real-time blood pressure has dropped significantly, but this occurs within the measurement interval so that approximately 15 minutes pass before this change in condition can be addressed by a clinician.

In the state of the art, to address the fact that the appropriate measurement interval varies depending upon patient condition, a clinician overseeing a patient will manually adjust the measurement interval in dependence upon their judgment of the acuity level of the patient and the risk of rapid deterioration of condition. The clinician uses standard protocols in combination with medical experience to set the best measurement interval.

It would be of benefit to provide a means for more objectively determining a best measurement time interval for a patient in view of the patient's condition.

SUMMARY OF THE INVENTION

The invention is defined by the claims.

According to examples in accordance with an aspect of the invention, there is provided a computer-implemented method. The method comprises receiving biological measurement data for a patient. The method further comprises applying a patient risk model to compute a risk parameter, p, indicative of a risk level of at least one pre-defined adverse clinical event, wherein at least one input to the model is the biological measurement data. The method further comprises obtaining a clinician-defined patient risk parameter, *A*. The method further comprises computing a measurement time interval, *T,* for successive non-invasive blood pressure measurements of the patient based on A and p and in accordance with the relationship:

$$T \propto \frac{A}{p^{\alpha}}$$

where a is pre-defined parameter, preferably having a value greater than zero.

The method further comprises controlling a non-invasive blood pressure measurement apparatus to acquire blood pressure measurements at a frequency defined by the determined time interval, *T.*

Thus embodiments of the invention provide a method of adaptively configuring the timing intervals between automatically triggered non-invasive blood pressure measurements based on a risk model which takes into account a current value of at least one biological parameter of the patient, and which advantageously also takes into account a clinician assessment of risk. Thus the clinician's guiding medical opinion about a situation still retains influence on the timing, but at the same time the time interval can be set in a partially objective manner, based on a risk model (configured to compute risk of at least one specific serious adverse event such as hypotension or hypertension).

The defined relationship for T keeps the product of the model-based probability for a critical event, p, and the blood pressure measurement interval time constant.

It will be appreciated that the patient typically might be at risk of more than one specific adverse clinician event and thus, in some embodiments, the model may be designed or trained to output a combined risk level of the patient suffering any of a set of different adverse events. Additionally or alternatively, the model may output a risk associated with only one adverse clinical event but wherein the method comprises running multiple versions of the model, each configured for predicting probability of a different type of adverse event, and selecting one the determined probability parameters based on a pre-defined criterion, or based on an input from the clinician.

By way of example, the at least one adverse or critical event may include hypotension and/or hypertension of the patient; a shock state of the patient, a cardiac arrest, or any cardiac event.

In some embodiments, the model is such that p is the probability of the occurrence of the event in a pre-defined time span.

In some embodiments, the biological measurement data includes real-time sensor data, or data derived therefrom.

In some embodiments, the biological measurement data includes historical biological measurement data for the patient retrieved from a datastore. By way of example, this might include heart rate or other vital signs, previous blood pressure measurement data, pathology tests or any other biological parameters.

In some embodiments, the risk parameter p is recalculated recurrently over a monitoring session for the patient. The biological measurement data is preferably updated for each recalculation.

In some embodiments, the risk model is a Bayesian model.

In some embodiments, the Bayesian model is a personalized risk model for the patient and/or a clinician treating the patient. In some embodiments, the Bayesian model is pre-configured in accordance with prior information including one or more of: patient medical history; patient condition severity/acuity; a training level of a clinician treating the patient; and a measure of a speed of physician reaction to condition changes.

In some embodiments, the method further comprises recurrently adjusting or updating the risk model based on a patient monitoring database comprising monitoring data for a plurality of patients. Thus, there can be implemented a self-learning model which can learn over time e.g. via retrospective analysis of monitored patients. This could be based on monitoring data acquired at a same medical institution or department, and/or could be based on monitoring data acquired more widely, for example population-wide across multiple different medical institutions.

In some embodiments, the biological measurement data includes data from one or more of: an Electro-cardiogram (ECG) sensing apparatus; a Photo-plethysmogram (PPG) sensing apparatus; a capnographic measurement apparatus; and a bioimpedance measurement apparatus.

The method can be implemented in software form.

Thus another aspect of this method is a computer program product comprising code means configured, when run on a processor, to cause the processor to perform a method in accordance with any embodiment or example in this disclosure.

The invention can also be embodied in hardware form. Thus another aspect of this method is a processing unit, comprising: an input/output; and one or more processors adapted to: receive at the input/output biological measurement data for a patient; apply a patient risk model to compute a risk parameter, p, indicative of a risk level of at least one pre-defined adverse clinical event, wherein at least one input to the model is the biological measurement data; obtain a clinician-defined patient risk parameter, $A$; compute a measurement time interval, $T$, for successive non-invasive blood pressure measurements of the patient based on A and p and in accordance with the relationship:

$$T \propto \frac{A}{p^{\alpha}}$$

where a is pre-defined parameter; and control, via generating control signals for output at the input/output, a non-invasive blood pressure measurement apparatus to acquire blood pressure measurements at a frequency defined by the determined time interval, T.

Another aspect of the invention is a system comprising the processing unit as outlined above or in accordance with any embodiment described in this disclosure; and a non-invasive blood pressure measurement apparatus operatively coupled with the processing unit.

In some embodiments, the system further comprises one or more biological parameter sensing devices, for acquiring biological measurement data, operatively coupled with the processing unit.

In some embodiments, the one or more biological parameter sensing devices include a PPG sensor integrated in a section of a non-invasive blood pressure measurement device.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a continuous blood pressure as a function of time for a patient in comparison with intermittent blood pressure measurements;
Fig. 2 outlines steps of an example method in accordance with one or more embodiments of the invention; and

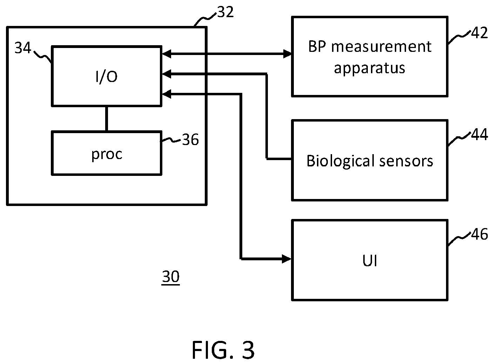

Fig. 3 schematically outlines components and a processing flow of an example processing arrangement in accordance with one or more embodiments of the invention.

DETAILED DESCRIPTION OF THE EMBODIMENTS

The invention will be described with reference to the Figures.
It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.
The invention provides a method for adaptively scheduling non-invasive blood pressure measurements with time intervals determined based on using a risk model to compute a risk of a patient suffering a pre-defined one or more adverse clinical events, for example within a pre-defined time window, and also based on a clinician risk assessment for a patient.
Blood pressure is typically measured via intermittent cuff non-invasive blood pressure (NIBP) measurements. Presently, medical staff must decide which blood pressure monitoring interval is appropriate based on an assessment of the hemodynamic stability of the patient.
Due to the potentially variable health status of a patient over time, the NIBP measurement intervals would ideally need continuous adaptation to reflect changing patient risk levels. However, in practical situations, this is not implemented, which can result in a number of possible adverse consequences, including:

- Missed critical events (e.g. hypo- and hypertensive phases);
- Unnecessary NIBP measurements, compromising patient comfort and causing sleep disturbance;
- Incorrect representation of patient response to a therapy (e.g. medication) with subsequent suboptimal treatment decisions

An example of NIBP trend monitoring over phases of instability and stability has already been discussed, with reference to Fig. 1. Fig. 1 illustrates that if NIBP monitoring is performed using intermittent measurements at fixed measurement time intervals (line 24), many episodes in which large BP changes (line 22) occur are missed. The selection of the measurement time interval by the clinician was not appropriated for this patient. It is noted that the illustrated graph of Fig. 1 shows the blood pressure trend line according to a zero-order hold representation (e.g. the discrete-time blood pressure signal is represented as a continuous-time signal by holding each sample value for one sample interval).
Blood pressure can be used as an indicator of a wide variety of medical adverse events in the body of the patient. Two adverse events of common concern for clinicians monitoring a patient are hypertension and hypotension phases. Hypertensive events are phases of very high blood pressure, typically understood to correspond to systolic blood pressure greater than about 140 mm/Hg. Hypotensive events are phases of very low blood pressure, typically understood to correspond to systolic blood pressure of less than about 90 mm/Hg.
Depending upon the phase of care, the probability of a patient death or longer-term health issues can increase significantly if time is spent in a hypotensive phase. For example, the post-operative phase of care in particular carries a significant risk of myocardial infarction and death even if only short amounts of time are spent in hypotensive phase, due to the resulting low perfusion (ischemia) of the heart and brain. Reference is made for example to the paper: McEvoy, M et al, Perioperative Quality Initiative consensus statement on postoperative blood pressure, risk and outcomes for elective surgery, British Journal of Anaesthesia, Volume 122, Issue 5, 2019, Pages 575-586. This paper explains, with reference to data presented on page 578, that each 10 minute episode of hypotension in post-operative day 0 is associated with a 3% increase in risk of myocardial infarction (MI) and death, and any episode at all of hypotension on post-operative days 1-4 are associated with an almost doubling of risk of MI and death.

If a patient is in hyper- or hypo-tensive phase, a clinician must act quickly through administration of medication or other interventions to bring the patient back into a normal hemodynamic state.

A hypotensive event starts at the moment the patient's true blood pressure value falls below a certain pre-defined critical threshold. The duration of the hypotensive blood pressure event can be defined as the interval time (minutes) from the moment when the actual blood pressure crosses below the critical blood pressure threshold to the moment it rises back above the pre-defined critical threshold.

The severity of the hypotension is related to its depth and duration. By depth is meant the lowest blood pressure value that is reached during the hypotensive episode. By generalizing this concept, a depth-duration dose metric (DDD) can be defined for a hypotension episode per patient in a general form as:

$$DDD_{\text{Hypotension Event}} = \sum \int_{t_1}^{t_2} (BP_{\text{Threshold}} - BP_{\text{Measured}}) dt$$

where $t_1$ is the staring time of the episode and $t_2$ is the stopping time of the episode, BPthreshold is the critical blood pressure threshold and $BP_{\text{measured}}$ is the patient's actual blood pressure value as a function of time. Reference may be made to the following paper which outlines this definition of DDD: D. W. Spaite et al., "Association of Out-of-Hospital Hypotension Depth and Duration With Traumatic Brain Injury Mortality," Ann. Emerg. Med., vol. 70, no. 4, pp. 522-530.e1, 2017.

With regards to the critical hypotension threshold, there are different ways in which this can be defined. Regardless of the definition, it has been shown in studies that in different populations (e.g. non-cardiac surgery patients and traumatic brain injury patients) that patient outcome is strongly dependent on the duration and depth (meaning how low the blood pressure drops, e.g. the minimum value) of the hypotensive episode. This is because damage is done cumulatively over the course of a continuous hypotensive episode. For example, by quantifying the hypotension dose (hypotension depth integrated across exposure time) of 7521 traumatic brain injury patients, it was shown in one study (*ibid*) that hypotension was associated with 20% increased mortality. Moreover, there is also evidence demonstrating that there exists a significant association between, on the one hand, the degree and duration of intra- and post- operative hypotension, and, on the other, myocardial injury and acute kidney injury (AKI). Reference is made for example to the paper: E. J. Mascha, et al, "Intraoperative Mean Arterial Pressure Variability and 30-day Mortality in Patients Having Noncardiac Surgery," Anesthesiology, vol. 123, no. 1, pp. 79-91, 2015.

More evidence demonstrating that the postoperative hypotension events are correlated with the poor outcome exist. One study showed that SBP < 90 mmHg was the most common cause (25%) for emergency team activation on a ward after orthopedic or general surgery. This study is detailed in the paper: S. Mohammed Iddrisu, et al, "Frequency, nature and timing of clinical deterioration in the early postoperative period," J. Clin. Nurs., vol. 27, no. 19-20, pp. 3544-3553, 2018.

A further study collected and studied data from 90 hospitals in the UK, Australia, and New Zealand and found that the most common antecedent event of patient deterioration was systolic blood pressure (SBP) < 90 mmHg. This study is detailed in the paper: J. Kause, et al, "A comparison of Antecedents to Cardiac Arrests, Deaths and Emergency Intensive care Admissions in Australia and New Zealand, and the United Kingdom - The ACADEMIA study," Resuscitation, vol. 62, no. 3, pp. 275-282, 2004.

As a consequence of these problems, the patient outcome is compromised, for example with longer hospital stays, higher morbidity and higher mortality. Therefore, improved blood pressure management would help to improve patient outcome and reduce complications, and also therefore also increase efficiency in hospitals.

Embodiments of the present invention provide a system and method to estimate and automatically configure optimal/appropriate blood pressure measurement intervals, personalized to a patient risk profile and physician opinion.

Fig. 1 outlines in block diagram form steps of an example method according to one or more embodiments. The steps will be recited in summary, before being explained further in the form of example embodiments.

The method comprises receiving 12 biological measurement data for a patient.

The method further comprises applying 14 a patient risk model to compute a risk parameter, p, indicative of a risk level of at least one pre-defined adverse clinical event, wherein at least one input to the model is the biological measurement data.

The method further comprises obtaining 16 a clinician-defined patient risk parameter, A.

The method further comprises computing 18 a measurement time interval, T, for successive non-invasive blood pressure measurements of the patient based on A and p and in accordance with the relationship

$$T \propto \frac{A}{p^\alpha}$$

where a is pre-defined parameter.

The method further comprises controlling 20 a non-invasive blood pressure measurement apparatus to acquire blood pressure measurements at a frequency defined by the determined time interval, *T*.

As noted above, the method can also be embodied in hardware form, for example in the form of a processing unit which is configured to carry out a method in accordance with any example or embodiment described in this document, or in accordance with any claim of this application.

To further aid understanding, Fig. 2 presents a schematic representation of an example processing unit 32 configured to execute a method in accordance with one or more embodiments of the invention, and also schematically showing the processing flow in more detail.

The processing unit 32 comprises an input/output 34 and one or more processors 36. The one or more processors are configured to perform the following steps:

receive at the input/output 34 biological measurement data for a patient, for example from one or more biological measurement devices or sensors 44;

apply a patient risk model to compute a risk parameter, p, indicative of a risk level of at least one pre-defined adverse clinical event, wherein at least one input to the model is the biological measurement data;

obtain a clinician-defined patient risk parameter, A, for example based on an input received at the input/output 34 from a user interface 46;

compute a measurement time interval, T, for successive non-invasive blood pressure measurements of the patient based on A and p and in accordance with the relationship

$$T \propto \frac{A}{p^\alpha}$$

where a is pre-defined parameter; and

control, via generating control signals for output at the input/output 34, a non-invasive blood pressure measurement apparatus 42 to acquire blood pressure measurements at a frequency defined by the determined time interval, T.

Another aspect of the invention is a system 30 which comprises the processing unit 32. Fig. 2 shows the processing unit 32 operating within the context of such a system. The system 30 may further comprise a non-invasive blood pressure measurement apparatus 42 operatively coupled with the processing unit, for example a cuff-based oscillometric blood pressure measurement apparatus. The system 30 may further comprise one or more biological parameter sensing devices 44, for acquiring biological measurement data, operatively coupled with the processing unit 32. By way of example, the one or more biological parameter sensing devices include a PPG sensor. A PPG sensor can be used to acquire pulse rate or heart rate, as well as blood oxygen concentration. It is also possible to infer a patient respiration rate from a PPG sensor signal. In some examples, the PPG sensor may advantageously be integrated in a section of a non-invasive blood pressure measurement device.

The system 30 may further comprise a user interface 46, for example for use in receiving a user-input indicative of the clinician-defined patient risk parameter, A, and/or for displaying a representation of the determined blood pressure measurement interval, a current value of the computed risk parameter, one or more biological measurements for the patient, or any combination thereof.

To further clarify, a brief sample workflow of the method in operation will now be outlined.

A patient in a medical institution is monitored with a set of one or more measurement devices 42, e.g. sensors, each configured to measure at least one biological parameter, e.g. a physiological parameter. Data outputs from the set of one or more measurement devices are received at the processing unit 32. This data forms a biological measurement data set for the patient. The processing unit could be a processing unit of a patient monitoring system in some examples. In addition to, or instead of, real-time data from measurement devices, the biological measurement data set may include data retrieved from a datastore which may include for example historical values for one or more biological parameters, or variables relating to the patient's medical history. It could include parameters which cannot be measured with sensors, such as pathology test results.

The processing unit 32 is further adapted to obtain an indication of a clinician-defined risk parameter, A. In some examples, this parameter may be obtained based on a user input received from a user interface device 46. For example, a clinician who is responsible for monitoring the patient may input the parameter. In another example, the parameter A may be determined for the patient based on a look-up table. For example a clinician may have determined in advance a set of relationships between different patient status classifications and associated values for the parameter A, and these may be encoded in a stored look-up table.

The processing unit 32 is further adapted to retrieve from a datastore a patient risk model. The patient risk model is adapted to receive input values for one or more variables corresponding to one or more respective biological measurements. These may include some or all of the values forming the received biological measurement dataset. The patient risk model is configured to generate as an output a value of a parameter, $p$, indicative of a risk level of at least one pre-defined adverse clinical event, such as hypotension, hypertension, or a cardiac event for instance. The value of p may be a probability value, and may take values ranging from 0 to 1. The model may be a generic model for any patient, or may be personalized to the patient, as will be explained in more detail later. The model could additionally or alternatively in some cases be personalized to the clinician monitoring the patient, as will also be explained in more detail later.

The processing unit 32, having retrieved the model, applies the model by supplying to it as input the relevant biological measurements, to thereby generate an output risk parameter value, p.

The processing unit 32 then computes a measurement time interval, T, for successive non-invasive blood pressure measurements of the patient based on first computing a value of $\frac{A}{p^{\alpha}}$, and computing a value of T based on the relationship $T \propto \frac{A}{p^{\alpha}}$, where a is further variable parameter.

In some embodiments, T may be determined simply by evaluating the equation $T = \frac{A}{p^{\alpha}}$.

In some embodiments, T may be determined based on evaluating the equation: $\mathrm{T} = \beta \frac{A}{p^{\alpha}}$, where $\beta$ may for example be a constant or $\beta$ may for example be a pre-defined transfer function. For example, $\beta$ may be a function of any or all of A, $p$, and $\alpha$.

The purpose of parameter $\beta$ is to provide a transfer or conversion of the value of $\frac{A}{p^{\alpha}}$ to a suitable value for T, in the correct units. The parameter $\beta$ can be pre-defined to provide the particular mapping between T and the ratio $\frac{A}{p^{\alpha}}$ which is desired.

The parameter a provides a further degree of freedom in the computation, for refining the computation of the time interval. The parameter in effect defines a relative weighting to be applied to the computer-determined risk parameter, $p$, as compared to the clinician-defined risk parameter, A. It will be recognized that as the value of a increases, so the scaling of the value of $p^{\alpha}$, as a function of $p$ increases in steepness. This has the effect that a higher value of a means more relative weight being placed on the computationally defined risk parameter $p$ as compared with the clinician defined risk parameter $A$.

The parameter a might in some examples be freely determined by a user. In some examples, it might be determined using a pre-defined lookup table or mapping which relates different patient or clinician statuses or classes to different values of a. For example, for a higher acuity patient, it might be preferred to set a lower value for a, since (for a value of p which varies between 0 and 1) this will have the effect of reducing the time intervals between blood pressure measurements if risk parameter $p$ increases during a time period between a clinician updating of their own assessment of risk, $A$. Therefore a higher value for a in these circumstances weighs toward greater caution. By way of further example, for a more experienced clinician who trusts their own assessment, the value of a might be set lower compared to the case of a less experienced clinician. This would have the effect (assuming a values of p which varies between 0 and 1) of giving higher relative weighting to the clinician-defined parameter A as compared to a case of a lower value of a.

The processing unit 32 may store in a memory a record of the newly calculated value of the computed interval time $T$. This may be updated each time $T$ is re-calculated. A log may be recorded of computed values of $T$ over time. $T$ may be re-calculated recurrently over a monitoring session for the patient. The risk parameter p may be recalculated recurrently over the monitoring session for the patient, with the biological measurement data being updated for each recalculation. Using the newly calculated value for p, the processing unit can recalculate $T$.

The processing unit 32 is further configured to control the NIBP measurement apparatus to acquire the BP measurements at a frequency defined by the determined time interval, T. The actual control of the NIBP measurement apparatus may be implemented by the processing unit 32 itself, or by a subsystem with which the processing unit is operatively coupled. In the latter case, the step of controlling the NIBP measurement apparatus to acquire the BP measurements at a frequency defined by the determined time interval may comprise simply communicating a control command to the NIBP measurement apparatus control subsystem to cause the subsystem to acquire the measurements at the computed interval T. Alternatively, if the processing unit is adapted to co-ordinate the control of the NIBP measurement apparatus itself, it may simply update a value of the parameter $T$ in a local register, and wherein the processing unit runs a control routine or program for triggering the BP measurements at time intervals determined by the current value of $T$ in the register.

As noted, it is preferable that the value of T be re-calculated recurrently. With regards to the timing of re-calculation, this could be performed at regular fixed time intervals, at intervals set by a clinician, or at intervals which are automatically determined or triggered. For example, one approach might be to re-calculate $T$ each time a new BP measurement is triggered. In other words, the value of $T$ itself is updated at a frequency determined by the current value of $T$, so that as each measurement interval ends, a new blood pressure measurement is acquired, and at the same time, a new value of the interval time $T$ is calculated. To calculate the new value of $T$, new values of each of the one or more biological measurements may be sampled, and these used as inputs for re-running the patient risk model to generate a new value for the parameter $p$, following which the new value of $T$ is computed using the relationship already outlined above.

It is noted that the patient risk model is naturally trained or programmed for modelling the patient risk for a specific adverse clinical event. Thus, in some embodiments, there may be multiple versions of the model, each configured for predicting probability of a different type of adverse event.

In some embodiments, a plurality of these versions of the risk model might be run, to derive a set of initial risk parameters $p_i$, each indicative of the probability of occurrence of a respective adverse clinical event. These initial risk parameters may then be further processed to derive the overall risk parameter $p$, indicative of risk of one or more adverse clinical events. In some embodiments, just one of the initial risk parameters, $p_i$, might be selected, corresponding to the adverse event which is most likely, or for which consequences for the patient are most severe. The selection may be based on a user input at the user interface. For instance, the user interface displays the set of computed initial risk parameters, along with an indication of the adverse clinical event to which each corresponds, and the user can select one to be used as the overall patient risk parameter. In another example, some or all parameters of the set of initial risk parameters may be combined to form the overall patient risk parameter, p. For instance, the probabilities may simply be added, to give a cumulative probability of any of the respective adverse clinical events occurring. Alternatively, an average might be computed.

Further details relating to one or more of the features outlined briefly above will now be described.

With regards to the biological measurement data, which is obtained as part of the method, it is preferred that this is received continuously, or in real-time, from one or more measurement devices or sensors arranged for monitoring the patient. This way, the patient risk model is provided with information which is up-to-date for the patient. The alternative approach would be to use archived biological measurement data for the patient.

By way of non-limiting example, the biological measurement data may include one or more of the following:

- Electrocardiogram data;
- photoplethysmogram (reflective or transmissive) data;
- pulse-oximetry / blood oxygen saturation data;
- capnography measurement data;
- bioimpedance measurement data;
- any other vital signs;
- pathology test results.

In some examples, the Photo-plethysmogram (PPG) sensor can be provided as an auxiliary sensor, separate to the blood pressure measurement apparatus, e.g. a finger PPG sensor. In some examples, the PPG sensor may be provided integrated in the blood pressure measurement apparatus, for example integrated in the blood pressure measurement cuff, and arranged such that, when the cuff is worn on a body part for measuring blood pressure, the PPG sensor optical area is in optical communication with a surface of the patient's skin.

These data can be received in real time from one or more biological parameter sensors. Additionally or alternatively, the biological measurement data can include historical biological measurement data for the patient retrieved from a datastore.

With regards to the non-invasive blood pressure (NIBP) measurement apparatus which is controlled as part of the method, this preferably comprises a cuff-based measurement device. The cuff might in use be applied to the patient's arm or wrist. A typical example of such a device operates by the cuff being actively pressurized e.g. with a pump or pressure reservoir, and the cuff pressure measured with a pressure sensor. Other ways to operate a cuff-based NIBP measurement device also exist and will be known to the skilled person. Any other type of NIBP measurement device can also be used as an alternative to a cuff-based system. NIBP measurement devices are very well understood in the field, and the skilled person will understand how to implement this feature.

A guiding insight behind the approach proposed by the inventors is the concept that, for a constant value of the clinician-defined parameter, A, a product of the probability, p (potentially adjusted by power laws), for a critical event and the NIBP interval time, T, be maintained constant. As a consequence, during any interval where A is left unchanged (i.e. the clinician does not provide any update of their own assessment of patient status or risk) an increased risk level of the patient, as computed by the model, results in a shorter NIBP interval.

As noted above, in accordance with this principle, in some embodiments, the value of the measurement time interval, *T*, could be determined as follows:

$$T = \frac{A}{p^{\alpha}}$$

*T is* the recommended measurement interval time. *A* is a physician-defined parameter, representing the physician's assessment of a risk level for a particular (pre-defined) adverse or critical event (e.g. hypotension, hypertension) of a patient for a coming monitoring period. It expected the physician would choose this in dependence upon their judgment of the situation, using their knowledge and experience to assess the risk level. The parameter, *p*, is the probability of the occurrence of the same pre-defined adverse event within a defined time window, as computed based on a patient risk model. The index/power parameter a can be adjusted so as to give more weight or less weight to the computer risk parameter, *p*, as compared to the clinician risk parameter A.

In some examples, a calibration multiplier or coefficient, β, may be added to the equation stated above so as to provide a correct mapping between the numerical value resulting from evaluation of $\frac{A}{p^{\alpha}}$ with the time units required by *T*. For example. The equation might then take the form $T = \beta \; \frac{A}{p^{\alpha}}$ . In some examples, β might be a single value constant. In some examples, β might be a function of any or all of A, p and a, so that it provides a transfer function between the value of $\frac{A}{p^{\alpha}}$ and suitable timing values *T* for the measurement intervals. Throughout this disclosure, reference to use of $\frac{A}{p^{\alpha}}$ alone can be understood to optionally include the coefficient or transfer function β as a further multiplier.

Alternatively, this calibration might be already incorporated into the allowable value ranges for A, p and a. For example, each of these parameters might take values across respective ranges, and/or at respective intervals across that range, which are configured such that evaluation of $\frac{A}{p^{\alpha}}$ automatically results in a value of T with the correct units.

As an example, the typical 15 min interval is estimated in a clinical scenario. Assuming a hospital policy allows, as a level of risk, an average of 1 event in 1000 patients with A = 0.001. The probability of an event per minute refers to p = 6.67* $10^{-5}$/ min resulting in a recommended interval time T = 15 min.

Purely by way of illustration, other and different example combinations with resulting interval times are shown in the table below. The parameter a has been set to a= 1.

| A | *p* | T [min] | Comment |
|---|---|---|---|
| 0.001 | 6.67* $10^{-5}$/ min | 15 | Initial interval time |
| 0.001 | 1* $10^{-3}$/ min | 1 | A higher probability p is computed by the model resulting in a lower interval time. |
| 0.001 | 1* $10^{-5}$ / min | 100 | A lower probability p is estimated yielding a higher interval time. |
| 0.002 | 6.67* $10^{-5}$ / min | 30 | Because of a different factor A the recommended interval is increased to 30 min |

This equation has the effect that an increased risk level of a patient potentially suffering a critical event results in a decreased measurement interval time.

The parameter A can be specified by a particular clinician treating a patient, representing the clinician's assessment of the risk level for the patient. Additionally or alternatively, the parameter A could be set based on a standardized guideline in a particular hospital department or based on generic guidelines, e.g. based on a lookup table relating different classifications of patient condition to different values for the parameter A.

A time-dependent estimation of the probability p of a critical event allows up to real-time adjustments of the interval time T.

In some embodiments, in addition to determining the value of T, the method may further comprise determining an estimate of an uncertainty of T. For this purpose, an uncertainty parameter $\Delta T$ may be computed.

A simple means for estimating an uncertainty is to define uncertainty interval values $\Delta A$ and $\Delta p$. $\Delta p$ can be estimated from the Bayesian model. The Bayesian model inherently outputs a probability distribution, along with an uncertainty dp, and this uncertainty can be used for $\Delta p$. The value for $\Delta A$ can be pre-defined for example using a look-up table, or defined according to a hospital policy in dependence upon patient condition or other factors.

Using $\Delta A$ and $\Delta p$, an estimated uncertainty parameter, $\Delta T$, for T could be computed as:

$$\Delta T = |\frac{1}{p^\alpha}|\Delta A + |(-\alpha)\frac{A}{p^{\alpha-1}}|\Delta p \ .$$

The computed value of the uncertainty parameter $\Delta T$ could be presented to the user (clinician) on a display of a user interface, along with the current computed value of T. The user could, via the user interface, be provided the option whether to accept T as the current measurement interval or not. The value of the uncertainty parameter provides additional information that assists the user in making this decision. If the uncertainty is high, then the user (clinician) might decide to lean toward caution and set the interval at a shorter time than the current value of T.

In some examples, the user could be provided a set of three preset options for the measurement interval time: T, T+$\Delta$T, or T-$\Delta$T. For example, if the uncertainty is relatively high, then the user might choose T-$\Delta$T.

In the above equation, the value of $\Delta A$ can be set to zero or estimated from historical data or from guidelines.

A part of the method is the use of a patient risk model to compute the risk parameter, p, indicative of a risk level of at least one pre-defined adverse clinical event. Details in respect of the patient risk model will now be discussed in further detail.

The patient risk model is configured such as to receive, in operation, inputs which comprise at least: values for each of one or more biological measurement parameters for the patient (discussed above). The patient risk model is configured to generate as output a value, p, indicative of probability of occurrence of a pre-defined adverse clinical event within a certain time interval.

A particularly well-suited approach to estimating a probability of this kind is to use a Bayesian statistical model.

A statistical model using Bayesian principles is able to determine a posterior probability distribution for a particular outcome, based on one or a plurality of input variables, wherein the model has been constructed based on one or a plurality of prior probability distributions for one or more events.

The use of Bayesian principles in this way to derive estimates of risk based on current values of one or more parameters and prior distributions for those parameters will be well known to the skilled person.

By way of example, a Bayesian model might be constructed such as to provide a value of *p* which indicates an estimated risk of blood pressure dropping below value *x* for y minutes, where x and y are defined values set so as to correspond to a hypotensive event of concern. For example, x might be 90 mm/Hg and y = 15 minutes. Prior distributions can be determined in advance for this scenario, as a function of variations in the measured biological parameters, d(t). If multiple biological parameters are to be measured and used as inputs to the model then prior distributions can be computed for *p* as a function of the multivariate space of **d**(t), where **d**(t) is a vector representing the set of all biological parameters (and/or other parameters) which are to be used as the inputs to the model.

Using the standard Bayes's theorem, the probability, *p*, of blood pressure (BP) dropping below x for y minutes can be expressed as: p (BP < x mmHg for y min | d(t),K) =

$$\frac{p \ (\ \mathbf{d}(t)\ |\ BP < x \ mmHg \ for \ y \ min, \ K) \ * \ p \ (BP < \ x \ mmHg \ for \ y \ min, \ K)}{p \ (\boldsymbol{d}(t))}$$

where:

K is a context parameter, e.g. patient condition; **d**(t) | BP < x mmHg for y min, K represents the probability distribution of obtaining measured dataset **d**(t) given that the condition [BP < x mmHg for y min; K] is true;

p (BP < x mmHg for y min, K) is the prior probability distribution of the condition [BP < x mmHg for y min; K] being true for any d(t); and

$p$ ($\mathbf{d}$(t)) is the prior probability distribution of obtaining $\mathbf{d}$(t) for any blood pressure measurement value.

Using standard Bayesian notation, the previously recited equation for T can be written as:

$$T = \frac{A}{\text{p (BP} < \text{x mmHg for y min, K)} \mid \mathbf{d}\text{ (t), K)}}$$

As explained above, the probability p can preferably be estimated recurrently, e.g. continuously or quasi-continuously, from acquired real-time biological measurement data and/or available historical data.

Using the Bayesian approach a wide range of different factors and variables can be taken into account so long as appropriate prior distributions are acquired. For example, in addition to measured biological measurement data $\mathbf{d}$(t), also parameters such as the following could be factored into the probability p which is output from the model:

- training level of a physician responsible for a patient;
- speed of a physician to react in a certain time span;
- patient history;
- patient severity.

Each of these variables could be codified according to a respective standardized score metric, so that prior distributions of $p$ can be measured over variable values of each.

By taking into account these various factors, the model can be effectively made to mimic the mental process of a human being taking into account new data.

In some embodiments, the method may further comprise updating the patient risk model based on monitoring data for patients in the medical institution. For example, monitoring data could include blood pressure measurement data and biological measurement data so that relationships between these parameters can be used in training the model. Thus, the model can learn over time via retrospective analysis of monitored patients.

Also, changes in clinician training level can be monitored and reflected in updates to the prior information used in training the model.

For updating the model, the method may further include a model update phase, which might be run once or at intervals which might be the same or different to the intervals for any (optional) updating of the parameters $p$ and $T$. For example, the update phase may include execution of an algorithm for obtaining and collating the relevant updated model prior distributions, and updating the model with the updated prior distributions.

It is noted that although embodiments described above employ use of a Bayesian model for the patient risk model, the risk model can be implemented using other types of models. For example, a machine learning model might be used in further examples comprising one or more machine learning algorithms trained to receive as input at least the biological measurement data for the patient and generate as output the risk parameter, $p$, indicative of a risk level of at least one pre-defined adverse clinical event. Suitable machine learning algorithms include, by way of non-limiting example, a multivariate regression model, or an artificial neural network such as a convolutional neural network.

It is noted that although embodiments described above relate to blood pressure measurement intervals, the concept of the invention is applicable more generally to determining time intervals for any other biological measurement, e.g. another physiological parameter measurement, e.g. another vital sign measurement. The method is most applicable for measurement modalities which cannot be, or it preferred should not be, performed continuously.

As mentioned previously, the invention can be embodied in software form. Thus another aspect of the invention is a computer program product comprising code means configured, when run on a processor, to cause the processor to perform a method in accordance with any example or embodiment of the invention described in this document, or in accordance with any claim of this patent application.

It is noted that for any embodiments of this invention, the method can be executed by a processing unit at any of a variety of different locations. For example, the method could be executed by a processing unit of a bed-side patient monitoring device, or by a patient monitoring system, or by a separate computing system, or by a cloud-based computing system.

Embodiments of the invention described above employ a processing unit. The processing unit may in general comprise a single processor or a plurality of processors. It may be located in a single containing device, structure or unit, or it may be distributed between a plurality of different devices, structures or units. Reference therefore to the processing unit being adapted or configured to perform a particular step or task may correspond to that step or task being performed by any one or more of a plurality of processing components, either alone or in combination. The skilled person will understand how such a distributed processing arrangement can be implemented. The processing unit may include a communication module or input/output for receiving data and outputting data to further components.

The one or more processors of the processing unit can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A computer-implemented method comprising:

   receiving biological measurement data for a patient;
   applying a patient risk model to compute a risk parameter, $p$, indicative of a risk level of at least one pre-defined adverse clinical event, wherein at least one input to the model is the biological measurement data;
   obtaining a clinician-defined patient risk parameter, $A$;
   computing a measurement time interval, $T$, for successive non-invasive blood pressure measurements of the patient based on A, and $p$, and in accordance with the relationship

   $$T \propto \frac{A}{p^\alpha}$$

   where a is pre-defined parameter; and
   controlling a non-invasive blood pressure measurement apparatus to acquire blood pressure measurements at a frequency defined by the determined time interval, $T$.

2. The method of claim 1, wherein the at least one adverse or critical event includes hypotension or hypertension of the patient.

3. The method of claim 1 or 2, wherein $p$ is the probability of the occurrence of the event in a defined time span.

4. The method of any of claims 1-3, wherein the biological measurement data includes real-time sensor data, or data derived therefrom.

5. The method of any of claims 1-4, wherein the biological measurement data includes historical biological measurement data for the patient retrieved from a datastore.

6. The method of any of claims 1-5, wherein the risk parameter *p* is recalculated recurrently over a monitoring session for the patient, with the biological measurement data being updated for each recalculation.

7. The method of any of claims 1-6, wherein the risk model is a Bayesian model.

8. The method of claim 7, wherein Bayesian model is a personalized risk model for the patient and/or a clinician treating the patient, and is pre-configured in accordance with prior information including one or more of:

   - patient medical history;
   - patient condition severity;
   - a training level of a clinician treating the patient;
   - a measure of a speed of physician reaction to condition changes.

9. The method of any of claims 1-8, further comprising recurrently adjusting or updating the risk model based on a patient monitoring database comprising monitoring data for a plurality of patients.

10. The method of any of claims 1-9, wherein the biological measurement data includes data from one or more of:

   an Electro-cardiogram (ECG) sensing apparatus;
   a Photo-plethysmogram (PPG) sensing apparatus;
   a capnographic measurement apparatus; and
   a bioimpedance measurement apparatus.

11. A computer program product comprising code means configured, when run on a processor, to cause the processor to perform a method in accordance with any of claims 1-10.

12. A processing unit, comprising:

   an input/output; and
   one or more processors adapted to:

      receive at the input/output biological measurement data for a patient;
      apply a patient risk model to compute a risk parameter, *p*, indicative of a risk level of at least one pre-defined adverse clinical event, wherein at least one input to the model is the biological measurement data;
      obtain a clinician-defined patient risk parameter, *A*;
      compute a measurement time interval, *T*, for successive non-invasive blood pressure measurements of the patient based on A, and *p*, and in accordance with the relationship

$$T \propto \frac{A}{p^\alpha}$$

      where a is pre-defined parameter; and
      control, via generating control signals for output at the input/output, a non-invasive blood pressure measurement apparatus to acquire blood pressure measurements at a frequency defined by the determined time interval, *T*.

13. A system comprising:

   the processing unit of claim 12; and
   a non-invasive blood pressure measurement apparatus operatively coupled with the processing unit.

14. The system of claim 13, further comprising one or more biological parameter sensing devices, for acquiring biological measurement data, operatively coupled with the processing unit.

15. The system of claim 14, wherein the one or more biological parameter sensing devices include a PPG sensor integrated in a section of a non-invasive blood pressure measurement device.

FIG. 1

FIG. 2

FIG. 3

Europäisches
Patentamt

European
Patent Office

Office européen
des brevets

**EUROPEAN SEARCH REPORT**

Application Number

EP 22 19 5047

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/042493 A1 (MUEHLSTEFF JENS [NL] ET AL) 15 February 2018 (2018-02-15) | 1-6,9-15 | INV. A61B5/022 |
| Y | * figures 13,14 * * paragraph [0032] * * paragraph [0041] – paragraph [0043] * * paragraph [0068] – paragraph [0071] * * paragraph [0082] * * paragraph [0104] * * paragraph [0110] – paragraph [0117] * ----- | 7,8 | A61B5/024 A61B5/00 G16H50/30 |
| Y | WO 2021/062292 A1 (ETIOMETRY INC [US]) 1 April 2021 (2021-04-01) | 7,8 | |
| A | * paragraph [00108] * * paragraph [00325] * ----- | 2,9 | |

TECHNICAL FIELDS
SEARCHED      (IPC)

A61B
G16H

The present search report has been drawn up for all claims

| Place of search | Date of completion of the search | Examiner |
|---|---|---|
| The Hague | 17 February 2023 | Oancea, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

**EP 22 19 5047**

This annex lists the patent family members relating to the patent documents cited in the above-mentioned European search report.
The members are as contained in the European Patent Office EDP file on
The European Patent Office is in no way liable for these particulars which are merely given for the purpose of information.

**17-02-2023**

| Patent document cited in search report | | Publication date | Patent family member(s) | | Publication date |
|---|---|---|---|---|---|
| US 2018042493 | A1 | 15-02-2018 | CN 107405088 | A | 28-11-2017 |
| | | | EP 3261526 | A1 | 03-01-2018 |
| | | | EP 3785618 | A1 | 03-03-2021 |
| | | | JP 6757734 | B2 | 23-09-2020 |
| | | | JP 7171665 | B2 | 15-11-2022 |
| | | | JP 2018510683 | A | 19-04-2018 |
| | | | JP 2020195813 | A | 10-12-2020 |
| | | | US 2018042493 | A1 | 15-02-2018 |
| | | | WO 2016135043 | A1 | 01-09-2016 |
| WO 2021062292 | A1 | 01-04-2021 | EP 4035178 | A1 | 03-08-2022 |
| | | | WO 2021062292 | A1 | 01-04-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MCEVOY, M et al.** Perioperative Quality Initiative consensus statement on postoperative blood pressure, risk and outcomes for elective surgery. *British Journal of Anaesthesia,* 2019, vol. 122 (5), 575-586
- **D. W. SPAITE et al.** Association of Out-of-Hospital Hypotension Depth and Duration With Traumatic Brain Injury Mortality. *Ann. Emerg. Med.,* 2017, vol. 70 (4), 522-530.e1
- **E. J. MASCHA et al.** Intraoperative Mean Arterial Pressure Variability and 30-day Mortality in Patients Having Noncardiac Surgery. *Anesthesiology,* 2015, vol. 123 (1), 79-91

- **S. MOHAMMED IDDRISU et al.** Frequency, nature and timing of clinical deterioration in the early postoperative period. *J. Clin. Nurs.,* 2018, vol. 27 (19-20), 3544-3553
- **J. KAUSE et al.** A comparison of Antecedents to Cardiac Arrests, Deaths and Emergency Intensive care Admissions in Australia and New Zealand, and the United Kingdom - The ACADEMIA study. *Resuscitation,* 2004, vol. 62 (3), 275-282